# EUROPEAN PATENT SPECIFICATION

(11) **EP 1 968 660 B1**
(45) Date of publication and mention of the grant of the patent: **24.11.2010**
(21) Application number: 06829121.0
(22) Date of filing: 24.11.2006
(51) Int. Cl.: A61L 27/58, A61F 2/24, A61F 2/06

(54) **BIODEGRADABLE SCAFFOLD**
BIOLOGISCH ABBAUBARES GERÜST
ECHAFAUDAGE BIODEGRADABLE

(30) Priority: 25.11.2005 GB 0524087
(43) Date of publication of application: 17.09.2008
(73) Proprietor: Universität Zürich, 8006 Zürich (CH)
(72) Inventor: HOERSTRUP, Simon, P., CH-8091 Zürich (CH); ZÜND, Gregor, CH-8091 Zürich (CH)
(74) Representative: Bremi, Tobias Hans
(86) International application number: PCT/EP2006/011303
(87) International publication number: WO 2007/059998

(56) References cited:
- WO-A-87/04627
- US-A- 4 923 380
- US-A- 6 126 686
- US-A1- 2003 138 945
- US-A1- 2005 085 898
- US-A1- 2005 240 262
- SONODA H, TAKAMIZAWA K, NAKAYAMA Y, YASUI H, MATSUDA T: "Small-diameter compliant arterial graft prosthesis: Design concept of coaxial double tubular graft and its fabrication." JOURNAL OF BIOMEDICAL MATERIALS RESEARCH, vol. 55, no. 3, 5 June 2001 (2001-06-05), pages 266-276, XP002463833

## Description

This invention relates to biodegradable scaffolds for prosthetic implants, in particular but not exclusively for vessel or valve implants. A particularly preferred embodiment of the present invention relates to a biodegradable scaffold configured for use as a scaffold for a prosthetic heart valve.

Many thousands of patients are treated every year for heart valve dysfunction. Of these patients, a significant number end up having one or more of their heart valves surgically replaced. In general terms, replacement valves are either mechanical or biological in nature, the latter being either xenografts or homografts. Xenografts comprise biological valve prostheses derived, for example, from porcine sources. Homografts include cryo-preserved or glutaraldehyde-fixed explanted human valves. All of these valve types have drawbacks.

For example, to avoid thromboembolic complications it is typically necessary for patients fitted with mechanical valve prostheses to be prescribed anticoagulation drugs for life, thereby permanently increasing that patient's risk of haemorrhaging. Infections are a further, often life-threatening complication for the patient. Biological xenografts, for example pig valves treated with glutaraldehyde, can be employed with good results but their tendency to calcify and hence degenerate sets a limit to their use.

Homografts, i.e. fixed heart valves isolated from human donors, are relatively resistant to infections, but are nonetheless exogenous tissue which can still cause immune reactions. It is also the case that the availability of homografts tends to be limited, and what's more such implants have a tendency to calcify and are therefore subject to considerable degeneration - typically requiring re-operation after 7 to 12 years.

One fundamental disadvantage which all of the above valve types share is intrinsic to their composition: they are all made of inorganic or fixed organic material and therefore lack the capacity for repair, for reconfiguration or for growth. As a result it is commonplace, particularly for younger patients, to have to undergo a series of operations as they grow up. Aside from the inherent risk associated with any surgical procedure, fusions occurring in the thorax following preceding operations tend to significantly increase the risks associated with each subsequent re-operation.

To alleviate problems such as these it has previously been proposed to "grow" autologous bioengineered prostheses for surgical implantation into patients. Such prostheses, being grown from the patient's own tissue, are less likely to provoke an adverse immune response. Importantly, as these implants are formed of living tissue, they are able to grow with the patient to such an extent that the need for re-operation (to replace prostheses that have reached the end of their life) is greatly reduced.

Our previous work has proposed an *in vitro* method whereby an autologous heart valve or prosthetic vessel can be grown in a bioreactor. In this method a suitable biodegradable support is incubated with homologous fibroblasts and/or myofibroblasts to form a connective tissue-like matrix that is then colonized with (typically autologous) endothelial cells. The connective tissue-like matrix is then transferred into a bioreactor for culturing and conditioning of the implant to the flow conditions that it is likely to experience when transplanted into the human body. The scaffold biodegrades (at least substantially) whilst in the bioreactor, so that at the time of its implantation, the prosthesis is at least substantially comprised of preferably autologous cell material that can readily be sewn into the receiving patient's heart. Further details of such prostheses are disclosed in European Patent No. 1077072

US 2003138945 discloses methods and systems for tissue engineering including an apparatus and methods for the growth, maintenance, and use of robust tissue sheets using tissue manipulation devices. The tissue manipulation devices provide a method of anchoring the tissue sheets to the cell culture substrate to promote prolonged maturation and subsequent increased mechanical strength. The tissue manipulation devices also provide a technique to facilitate removal of the sheet from the culture container and subsequent production steps to assemble more complex three dimensional organs from the robust sheet. The disclosure provides methods and composition derived from robust human sheets to build tissue engineered blood vessels, heart valves, stents, and endarterectomy patches and the like. The use of these tissue manipulation devices facilitates the assembly of complex tissue engineered organs using the novel sheet-based tissue engineering approach.

US 6126686 discloses a method for preparing vascular valves from submucosal tissue. Both bicuspid and tricuspid valve constructs are described. The bicuspid constructs can be formed with or without a supporting stent. The tricuspid constructs are formed by fixing submucosal tissue to a supporting stent, folding the submucosal tissue, and forming the valve commissures from the folded submucosal tissue by cutting along the folds. The artificial vascular valves are useful for replacing damaged or diseased valves of a warm-blooded vertebrate.

The scaffold disclosed in this application takes one of two forms. For a prosthetic vessel the two longer sides of a generally rectangular piece of biodegradable scaffold material are overlapped and secured together, typically by suturing, to form a tubular scaffold structure for subsequent incubation with homologous fibroblasts and/or myofibroblasts. For a heart valve, this tubular scaffold structure is adapted by inserting a ring of biomaterial the upper side of which is shaped into three symmetric and concentric leaflets. The three leaflets overlap at their unsecured edges to mimic the structure of a natural heart valve, and can move to open and close the lumen of the tubular structure.

Whilst the techniques we have previously proposed have proven to be of great utility, both for valve prostheses and vessel prostheses, we have identified several aspects of the scaffolds previously proposed which could merit some improvement.

For example, whilst the tubular structure previously proposed functions adequately for colonisation and subsequent conditioning in a bioreactor, feedback from surgeons using the implants has indicated that a significant proportion of those surgeons are of the view that the implants are overly delicate. Whilst the implants are

of course sufficiently strong to function as designed, the perception that the devices are overly delicate has been identified as an area for improvement, and it has been suggested that this perception may be due to surgeons generally preferring to implant devices that are more solid in construction, perhaps due to a misconception that more solidly constructed devices are more likely to last when implanted in a patient.

It is also the case that as the devices are relatively delicate, the surgeons must be more careful not to damage the device when suturing it in place in a patient than they would otherwise have to be with a more robust implant. Any such damage may lead to a device being unsuitable for implantation, or more worryingly may provide regions of weakness in the device that might tear when the device is implanted and exposed to the flow conditions prevailing in the human or animal body.

As described above, previous valve production techniques have involved suturing a ring of biomaterial into a vessel prosthesis (the upper side of the biomaterial having been shaped into three symmetric and concentric leaflets) so as to form a structure which mimics the natural structure of a heart valve. Whilst this arrangement functions adequately, it is an extremely delicate and hence arduous task to suture the biomaterial in place without damaging the tubular structure.

It is also the case that as the sutures are components of the device that cannot be colonised with cells, they are components on which the connective tissue structure that provides the device with its mechanical strength cannot form. The effect of this is that the sutures each form - at least theoretically - a point of weakness in the device, at each of which the device has an increased risk of mechanical failure. As any mechanical failure that occurs following implantation could potentially prove fatal to the patient, it would be highly beneficial if a device structure could be devised that did not inherently include regions of lesser mechanical strength - at least in critical regions such as the join between the leaflets and the peripheral wall of the tubular body.

A further disadvantage associated with the manufacturing techniques previously proposed is that reproducibility is extremely difficult. Specifically, it is very hard for any two implants to be constructed, within specified tolerances, such that those two devices are identical. Hitherto each and every valve scaffold has been hand fabricated, and it would be greatly advantageous if a scaffold design could be devised that facilitated scaffold construction.

Embodiments of the present invention have been conceived with the aim of alleviating one or more of these drawbacks, and to this end a presently preferred embodiment of the present invention provides a biodegradable scaffold for a prosthetic implant, the scaffold comprising: first and second tubular scaffold components co-axially arranged one inside the other and secured one to the other, each said tubular scaffold component being fabricated from a sheet of biodegradable material having two generally parallel peripheral edges that are overlapped and secured one to the other to form a tube. Providing two tubular scaffold components, secured one to the other, is advantageous in comparison with the previously proposed single tube as the double tube arrangement herein proposed is significantly stiffer and stronger, and as such is less likely to be perceived as being flimsy and generally unsuitable for implantation.

The first and second tubular scaffold components are arranged such that the overlapped peripheral secured edges of said first tubular scaffold component and said second tubular scaffold component are at different positions about the circumference of the biodegradable scaffold. This arrangement is particularly advantageous if the technique selected to secure the overlapped peripheral edges to form the first and second tubular scaffold components results in the creation of one or more regions that are unsuitable for colonisation with fibroblasts and/or myofibroblasts to form a connective tissue structure. The reason for this is that by avoiding having the join of the first tubular component at least substantially coincident with the join of the second, it is possible to avoid generating a region of potential structural weakness that runs all of the way through the peripheral wall of the implant. In a highly preferred arrangement, the secured overlapped regions of the first and second tubular components are arranged to be substantially diametrically opposite one another about the circumference of said biodegradable scaffold. In such an arrangement, a mechanical failure at one or other of the overlapped regions should not result in a catastrophic failure of the device itself.

In one embodiment of the invention the overlapped peripheral edges of at least one of said tubular scaffold components may be sutured one to the other. In another embodiment, the overlapped peripheral edges of at least one of said tubular scaffold components may be joined one to the other by applying heat to weld one said edge to the other. In either embodiment one said edge may be joined to the other along substantially the entire length of the peripheral edges. Alternatively, one said edge may be joined to the other at one or more discrete locations.

In another embodiment the overlapped peripheral edges of at least one of said tubular scaffold components may be joined by passing a member (such as a needle) having a roughened outer surface through the overlapped peripheral edges, the roughened outer surface drawing fibres of one said overlapped edge into the other to knit the one to the other.

In the preferred arrangement, said first and second tubular components are secured one to the other at a plurality of locations about the periphery of the biodegradable scaffold.

In a further embodiment of the present invention, the biodegradable scaffold may comprise a third tubular scaffold component coaxially arranged with and overlying said first and second tubular components, wherein said third tubular scaffold component is fabricated from a sheet of biodegradable material having two generally parallel peripheral edges that are overlapped and secured one to the other to form a tube, and is secured to at least one of said first and second tubular scaffold components. In the preferred arrangement, the third tubular scaffold component is secured to at least one of said first and second tubular scaffold components at a plurality of discrete locations about the periphery of the biodegradable scaffold.

In a highly preferred arrangement, the plurality of discrete locations at which said third tubular scaffold component is secured to at least one of said first and second tubular scaffold components is different to the plurality of discrete locations at which said first and second tubular components are secured one to the other. By virtue of this arrangement it is possible to avoid overlying the locations at which the first and second tubular components are secured one to the other with the locations at which the third tubular component is secured to at least one of the first and second components, and hence it is possible to avoid generating regions of potential structural weakness running all of the way through the implant.

Another embodiment of the present invention provides a biodegradable scaffold for a heart valve prosthesis, the scaffold comprising coaxially arranged inner, middle and outer tubular scaffold components each of which is fabricated from a sheet of biodegradable material having two generally parallel peripheral edges that are overlapped and secured one to the other to form a tube; wherein said inner scaffold component comprises a plurality of peaks at each of which the scaffold component has a larger longitudinal dimension in an axial direction, said peaks being separated from one another by trough regions wherein the scaffold component has a smaller longitudinal dimension in said axial direction, and a distal end of said middle scaffold component is shaped about said inner scaffold component to provide a plurality of valve leaflets each of which is generally coincident with respective ones of said troughs.

Yet another embodiment relates to a method of assembling a biodegradable scaffold, the method comprising wrapping a first sheet of biodegradable material round a jig component, securing overlapped parallel ends of said first sheet together to form a first tubular scaffold component, wrapping a second sheet of biodegradable material round said first tubular scaffold component, and securing overlapped parallel ends of said second sheet together to form a second tubular scaffold component coaxially arranged with said first tubular component.

Another embodiment of the present invention relates to a method of assembling a biodegradable scaffold, the method comprising: wrapping a first sheet of biodegradable material round a jig component, said jig component having a crown-like shape consisting of a plurality of peaks at each of which the jig component has a larger longitudinal dimension in an axial direction, said peaks being separated from one another by trough regions wherein the jig component has a smaller longitudinal dimension in said axial direction; securing overlapped parallel ends of said first sheet together to form a first tubular scaffold component; conforming said first sheet of material to the crown-like shape of said jig component; wrapping a second sheet of biodegradable material round said first tubular scaffold component, and securing overlapped parallel ends of said second sheet together to form a second tubular scaffold component coaxially arranged with said first tubular component.

Additional features and advantages of these and other preferred aspects of the present invention are set out in the accompanying claims and elsewhere in the following detailed description.

A number of preferred embodiments of the present invention will now be described, by way of illustrative example only, with reference to the accompanying drawings, in which:
Fig. 1 is a perspective view of a vessel scaffold in accordance with a preferred embodiment of the present invention;
Fig. 2 is a top perspective view of a valve scaffold in accordance with another embodiment of the present invention;
Fig. 3 is another top perspective view of the scaffold of Fig. 2 with part thereof shown in ghost;
Fig. 4 is a perspective view of the bottom of the scaffold depicted in Fig. 3;

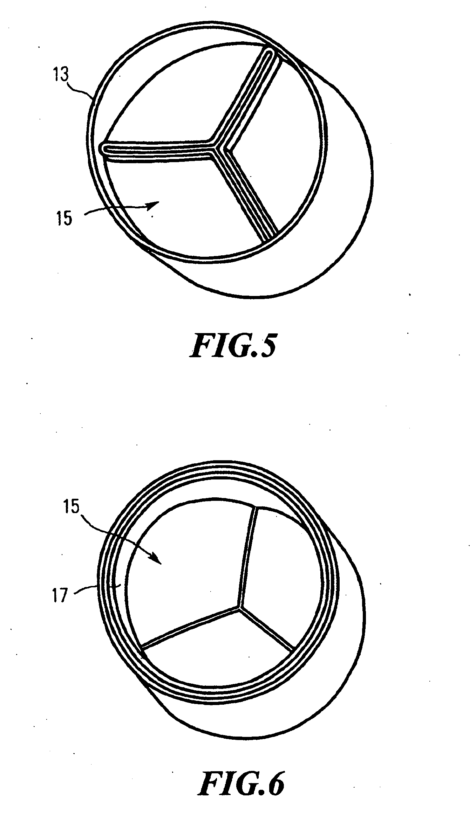

Fig. 5 is another top perspective view of the scaffold of Fig. 2;
Fig. 6 is another perspective view of the bottom of the scaffold depicted in Fig. 3;
Fig. 7 is a perspective view of a first (inner) tubular scaffold component;
Fig. 8 is a perspective view of a second (middle) tubular scaffold component;
Fig. 9 is a perspective view, partly in ghost, showing the first and second scaffold components of Figs. 7 and 8 respectively assembled together,
Fig. 10 is a perspective view of a third (outer) tubular scaffold component;
Fig. 11 is a perspective view of part of a jig set particularly useful for fashioning the component depicted in Fig. 7;
Fig. 12 is a cross-sectional view of the jig assembly shown in Fig. 11;
Fig. 13 is a perspective view of another part of the jig set;
Fig. 14 is a perspective view of a plunger for use with the jig set; and
Figs. 15 and 16 are perspective and cross-sectional views, respectively, of several assembled jig set components.

Various presently preferred embodiments of the present invention will now be described in detail. However, prior to embarking upon that detailed description, it is useful at this juncture to devote a few paragraphs to an illustration of the different materials from which the scaffold may be constructed.

This explanation should not be taken as an exhaustive list of possible materials, as further suitable materials will be immediately apparent to persons of ordinary skill in the art.

In one example, the biodegradable scaffold could be of sheets of material that are built up from polymer fibres around a porous polymer core or an acellular biological tissue. Suitable synthetic polymers include bioerodable polymers, such as polyglycolic acid (PGA), polylactic acid (PLA), polyhydroxyalkanoate (PHA) and poly-4-hydroxybutyrate (P4HB), polycaprolactones, poly(lactide-co-glycolide) (PLGA), polycarbonates, polyamides, polyanhydrides, polyamino acids, polyorthoesters, polyacetates, polycyanoacrylates and degradable polyurethanes, and non-erodable polymers, such as polyacrylates, ethylene/vinyl acetate polymers and other substituted cellulose acetates and derivatives thereof. Polyesters are particularly preferred.

Additional biodegradable polymers include polymers such as polyesters of hydroxycarboxy acids, polyanhydrides of dicarboxy esters and copolymers of hydroxycarboxy acids and dicarboxy esters. In another example, the scaffold material could be of a synthetic polymer of at least one of the following monomers: glycolide, lactide, p-dioxanone, caprolactone, trimethylene carbonate and butyrolactone. The material could also be chosen from a group consisting of polymers or copolymers of glycolic acid, lactic acid and sebacic acid, and polyglycolic acid polymers have proven to be particularly suitable.

Polymers of the type aforementioned can be used either in the pure form, in mixtures of two or more of the substances mentioned, or mixtures of these substances and further biodegradable polymers. In a particularly preferred arrangement, a copolymer of 85 % PGA and 15 % PLA is used.

Alternatively the scaffold material could include a polyhydroxyalkanoate (PHA), and this PHA could be coated with a further relatively non-degradable polymer. In this case a PHA that degrades *in vivo* within less than 9 months, even more preferably in less than 6 months and most preferably in less than 3 months, is preferred. As examples, the PHA may comprise 2-, 3-, 4- or 5-hydroxy acids, e.g. poly-4-hydroxybutyrates, or a poly-4-hydroxybutyrate-co-3-hydroxybutyrate and combinations thereof. Poly-4-hydroxybutyrate has proven to be particularly suitable.

The scaffold material may also comprise homopolymers and copolymers with any desired combination of the following monomers: 3-hydroxybutyrates, 3-hydroxyvalerate, 3-hydroxypropionate, 2-hydroxybutyrate, 4-hydroxybutyrate, 4-hydroxyvalerate, 3-hydroxyhexanoate, 3-hydroxyheptanoate, 3-hydroxyoctanoate, 3-hydroxynonanoate, 3-hydroxytridecanoate, 3-hydroxytetradecanoate, 3-hydroxypentadecanoate, 3-hydroxyhexadecanoate, 3-hydroxyheptadecanoate and 3-hydroxyoctadecanoate.

In general terms, it is preferred for the biodegradable scaffold to have a polymer density of 40 to 120 mg/cm³. The reason for this is that below 40 mg/cm³ the polymer fabric is too unstable, and above 120 mg/cm³ the material is too dense to allow colonisation within an acceptable period of time. In general terms a density of 50 to 80 mg/cm³, for example 70 mg/cm³, has proven to be particularly effective. A polymeric support from Albany International Research, Mensville, MA, USA having a density of approx. 70 mg/cm³ has been tested, as well as polymeric supports from TRANSOME INC., Palm Bay, FL. USA, and ITV Denkendorf, Koerschtalstrasse 26, 73770 Denkendorf, Germany.

In general terms, it is preferred for the fibres of the scaffold material to have a diameter in the range of 6 to 20 µm, preferably 10 to 18 µm. Fabrics having other fibre thicknesses may alternatively be employed so long as the fibres are sufficient thick to impart a measure of stability to the support, and not so thick as to prevent colonization of the support with fibroblasts and/or myofibroblasts. Pore sizes of 80 - 240 µm have proved favourable, and such pores can be achieved by the socalled (and well known) salt leaching technique.

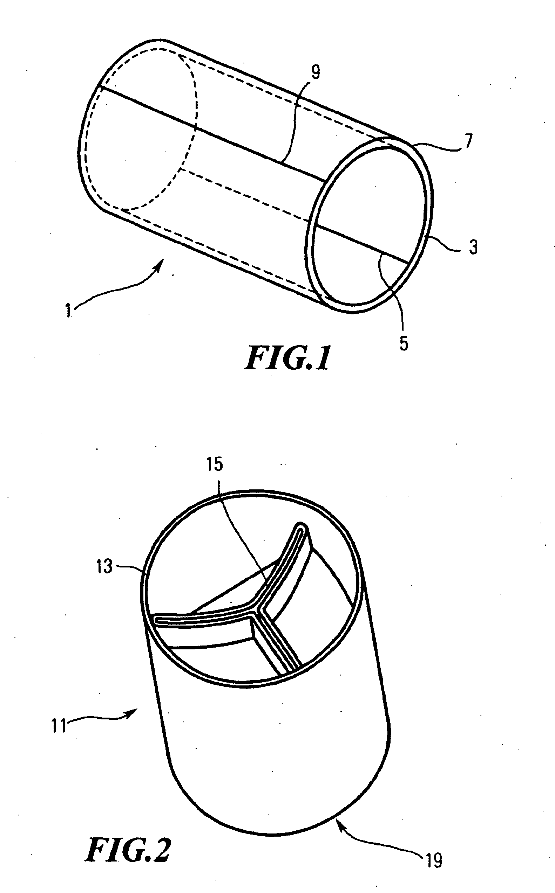

Referring now to Fig. 1 of the accompanying drawings, there is shown a biodegradable scaffold I that is configured, in this example, as a vessel prosthesis.

The scaffold 1 consists of a first tubular scaffold component 3 that is formed from a sheet of biodegradable material that has two generally parallel peripheral edges which are overlapped and secured, for example along join line 5, one to the other to form a tube.

The first tubular scaffold component 3 is arranged co-axially within a second tubular scaffold component 7. This second component is, in common with the first component, formed from a sheet of biodegradable material that has two generally parallel peripheral edges which are overlapped and secured, for example along join line 9, one to the other to form a tube.

As will be appreciated, the tubes can be independently formed and then inserted one within the other, or alternatively the first tube can be formed and then the second can be formed around the first.

Once the tubes are arranged one inside the other, the first and second tubes are joined together. This may be accomplished by suturing, welding or gluing the two tubes together. The tubes may be joined at a plurality of discrete locations evenly spaced over the periphery of the scaffold, or alternatively they could be joined circumferentially along respective circumferential lines spaced from either end of the scaffold.

As welded, sutured or glued regions cannot be colonised (at least to a sufficient extent) the points at which the tubes are joined to be spaced from one another to thereby avoid generating lines of structural weakness through the prosthesis. For this reason it is also preferred that the tubes be arranged one inside the other such that the join lines 5 and 9 do not coincide, and preferably are radially well spaced from one another (for example generally diametrically opposed to one another as shown in Fig. 1).

As an alternative to joining the tubes by suturing, welding or gluing, the first and second tubes could be joined (when forming the tubes and/or when joining one tube to the other) by passing an implement, such as a needle, with a roughened outer surface back and forth through the material of the scaffold. This arrangement secures the tubes together by drawing fibres from the material of one tube into the other to knit the tubes together, and is advantageous as it avoids the creation of regions where colonisation to the required extent is not possible.

Once the scaffold has been constructed and sterilised, it can then be incubated with homologous fibroblasts and/or myofibroblasts to form a connective tissue-like matrix that is then colonised with (typically autologous) endothelial cells. The colonised connective tissue-like matrix is then transferred into a bioreactor for culturing and conditioning of the implant to the flow conditions that it is likely to experience when transplanted into the human body. The bioreactor may comprise, in a preferred arrangement, part of a bioreactor system of the type described in a copending Patent Application entitled "BIOREACTOR SYSTEM" and bearing publication number WO 2007059999
A vessel prosthesis of the type depicted in Fig. 1 is comprised, as shown, of two layers of biodegradable material, and as such is significantly more robust (i.e. less flimsy) than vessel prostheses of the type previously disclosed. Furthermore, by encapsulating one tube within another, it is possible to avoid or at least mitigate the harm that might otherwise be caused if a single layer prosthesis of the type previously disclosed were to burst at a point of weakness in the body of the prosthesis. It is also the case that attaching the tubes by means of a roughened implement is highly advantageous as such an arrangement avoids forming regions on the prosthesis where the formation of a connective tissue-like matrix is impaired.

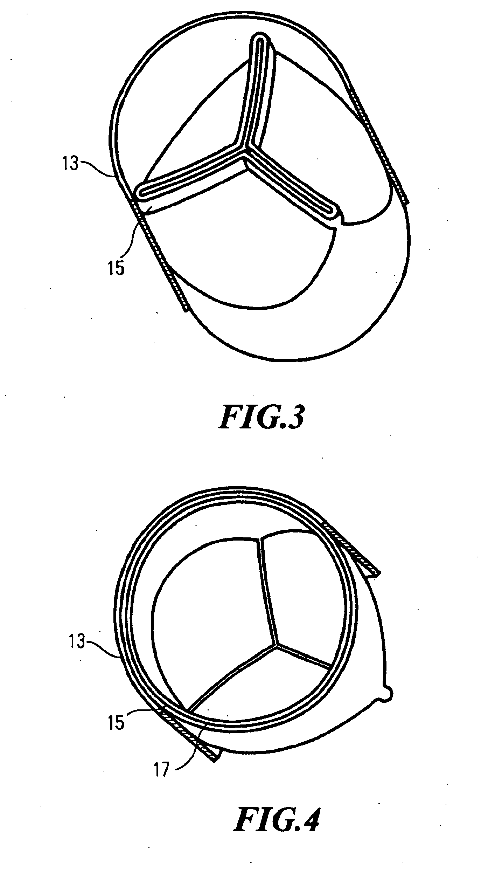

Referring now to Figs. 2 and 3 of the accompanying drawings, there are depicted top perspective views of a scaffold 11 according to another embodiment of the present invention.

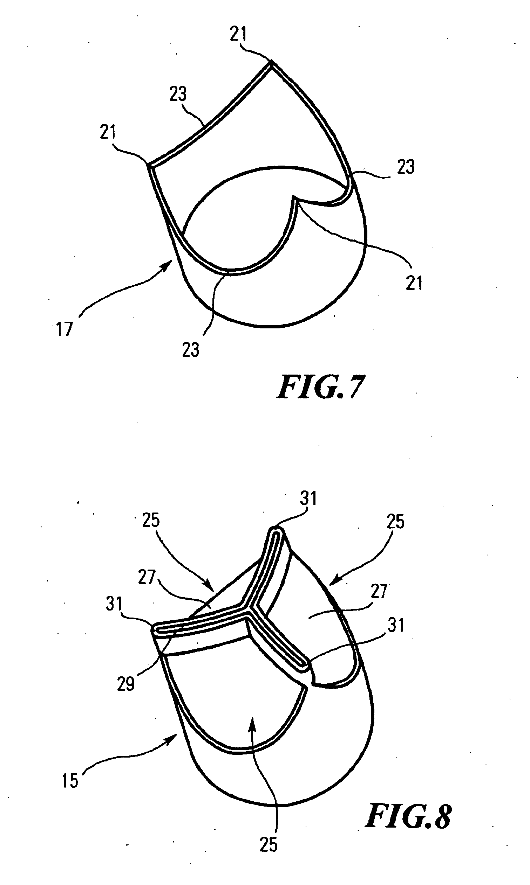

The scaffold 11 of this embodiment functions as a valve prosthesis and is comprised of three tubular scaffold components, an outer (third) component 13, a middle (second) component 15 that is shaped to function as valve leaflets, and an inner (first) component 17 (see Figs. 4 and 7) which functions to reinforce a proximal end 19 of the scaffold 11.

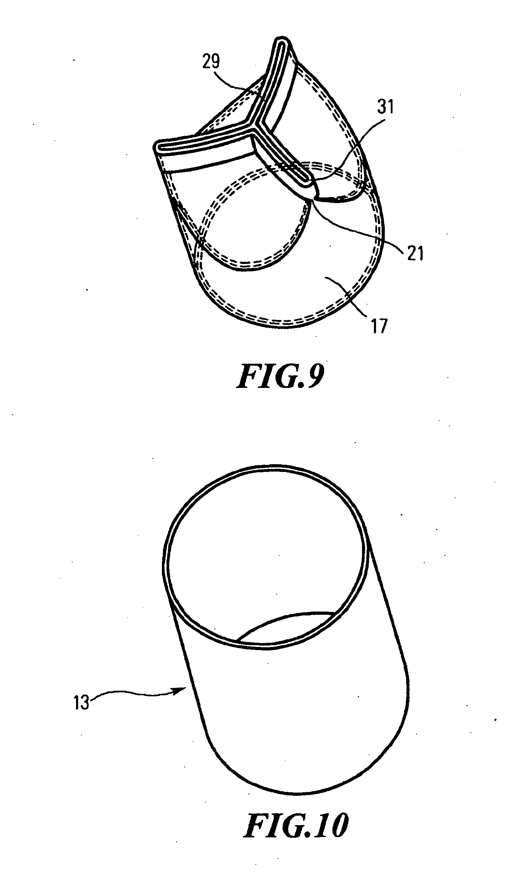

As shown in Fig. 10, the outer component 13 is a tubular scaffold component consisting of a piece of scaffold material with two parallel sides that have been overlapped and joined one to the other to form a tube.

As shown in Fig. 7, the inner component 17 is a tubular scaffold component that has been cut or otherwise formed to include a plurality (in this instance three) peaks 21 which are separated by troughs 23, the peaks and troughs giving the inner component the general appearance of a crown. The crown shaped inner component includes, as will later become apparent, as many peaks as there are valve leaflets formed by shaping the middle component 15.

Referring now to Fig. 8, the middle component 15 is a tubular scaffold component that includes, in this instance, three regions 25 that have been deformed into the lumen of the middle component to each form a valve leaflet 27 that abuts to close the valve with neighbouring leaflets. As shown in Fig. 9, the middle component 15 (shown in ghost) is coaxially arranged with the inner component 17, and the regions 25 deformed to form the leaflets of the valve coincide with the troughs 23 in the inner component 17.

As will now be apparent, when fluid is pumped into the proximal end 19 of the scaffold 11 (as happens when the scaffold is mounted in a bioreactor for conditioning), the applied fluid pressure causes the regions 27 forming the leaflets to open along a generally Y-shaped line 29 extending from a central axis of the second component lumen to points 31 that coincide with the peaks 21 of the first component. 17, and thereby open the valve to fluid flow. Between pump cycles, the fluid backpressure causes the leaflets to return to their closed position (as depicted in Fig. 8, for example) to once more close the valve to fluid flow.

To maintain the structural integrity of the scaffold, the inner and middle tubular components are secured to one another with the inner component coaxially arranged in the lumen of the middle component. As with the previous embodiment, the inner and middle components may be glued, sutured, welded or knitted to one another. The outer component is secured to the middle component, or optionally to both the middle and inner components again by any of the methods previously described. Once again, the inner, middle and outer components are arranged such that the overlapped regions thereof (which are joined to create each of the tubular components) do not coincide, and are preferably widely spaced (in this instance by roughly 120 degrees).

In a highly preferred arrangement the inner and middle components are secured to one another in a plurality of positions repeated in a pattern that extends round the circumference of the middle component. To avoid generating regions of structural weakness, particularly when the method chosen to secure the middle and inner components creates regions that are less suitable for incubation, the outer component is secured to the middle or the middle and inner components in a second pattern, different from the first mentioned pattern. By virtue of this arrangement it is possible to avoid creating regions of potential structural weakness that extend all the way through the scaffold structure.

It is also highly preferred for the middle component to be secured to the inner component along the edge of each leaflet 27 (corresponding to the edge of each trough 23) so as to provide greater mechanical strength to the region of the valve that is likely to suffer the largest amount of mechanical stress.

It will be appreciated that the valve scaffold of this embodiment has several advantages over previously proposed valve scaffolds. Firstly, as the scaffold is comprised, at least in part, of three layers of material it is significantly more robust than previously proposed scaffolds, and as such is less likely to be disregarded as being too delicate or flimsy. Secondly, the scaffold of this embodiment has a greater mechanical strength, particularly in the proximal end thereof, and this increased mechanical strength reduces the likelihood of the scaffold, and ultimately the prosthesis, failing. A third advantage of the scaffold of this embodiment is that it is significantly easier to manufacture than scaffolds of the type previously proposed which employed discrete leaflets sutured to a vessel,

As has been mentioned above, a significant drawback associated with previously proposed devices is that those devices are not easily reproducible. As will now be described in detail, an associated aspect of the present invention provides a jig set and a method of assembly that greatly facilitates the manufacture of a scaffold of the type disclosed.

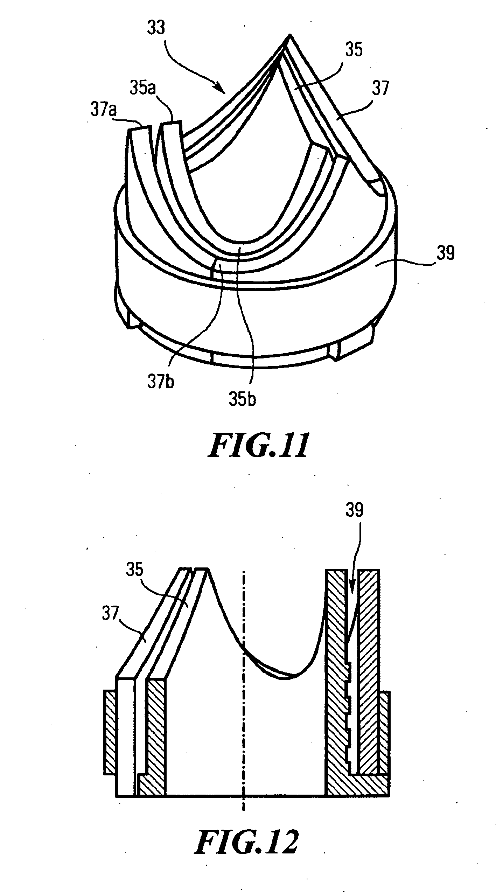

The first stage in the scaffold assembly process comprises the formation of the inner component 17. This is accomplished with a jig assembly 33 as depicted schematically in Figs. 11 and 12 (Fig. 12 being a sectional view of the jig depicted in Fig. 11).

The jig assembly 33 comprises an inner crown jig 35, an outer crown jig 37 and a retaining ring 39. As depicted in Fig. 11, the outer crown jig 37 fits over the inner jig 35, and in the preferred arrangement the inner jig 35 locates in the peripheral wall of the outer jig 37 to prevent relative movement between the two jig components. Each of the inner and outer jigs are provided with peaks and troughs, 35a, 37a and 35b, 37b respectively, which are shaped to be identical to those of the inner component 17.

To fashion the inner component a piece of scaffold material (not shown) is wrapped around the periphery of the inner jig 35, the adjacent parallel edges are overlapped (as described previously) and then secured one to the other. The outer jig 37 is then fitted over the inner jig 35, and then retaining ring is fitted over the outer jig 37 to hold the inner and outer jigs together with the scaffold material closely received in a gap 39 between them. At this stage the piece of scaffold material extends beyond the inner and outer jig troughs 35b, 37b, and most probably above the peaks 35a, 37a.

To form the inner component, a scalpel or other cutting device is used to cut the scaffold material into the form depicted in Fig. 7 using the inner and outer jigs 35, 37 as a guide.

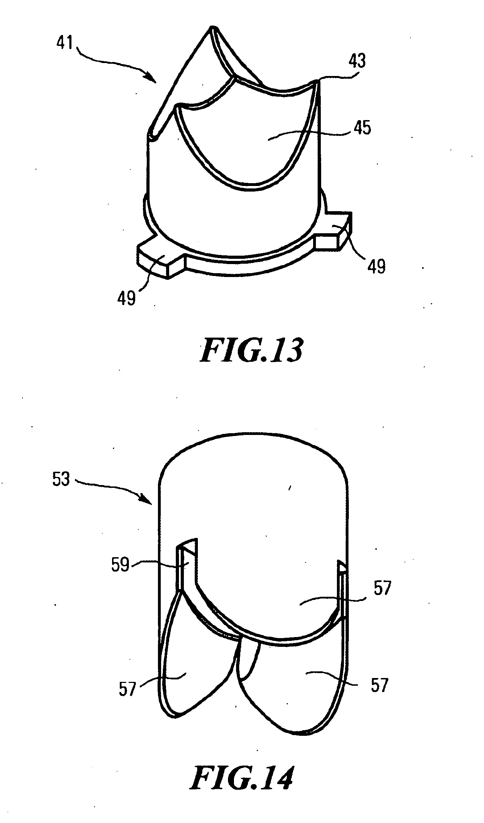

Once the inner scaffold component has been cut, the retaining ring 39 and outer jig 37 are removed and the inner scaffold component is transferred to a middle component jig 41 depicted schematically in Fig. 13. This middle component jig comprises a number of peaks 43 and intervening valleys 45, and once the inner scaffold component is located on the middle component jig it should be carefully aligned such that the peaks of the inner scaffold component are aligned with those of the middle component jig.

Once the inner scaffold component has been correctly aligned on the middle component jig 41, a second piece of scaffold material is secured round the inner scaffold component and the middle component jig, and is formed into a tube (by overlapping and securing as previously described) that is sufficiently long to enable the second piece of scaffold material to be deformed to form the valve leaflets in the manner described below.

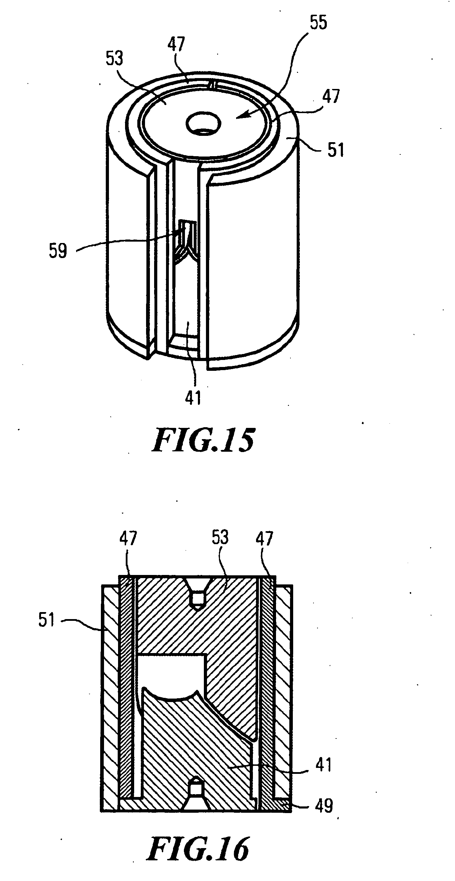

Next a pair of generally semi-circular (in cross-section) retaining members 47 (shown in Figs 15 and 16) are fitted around the second piece of scaffold material (and the inner scaffold component and middle component jig 41 provided therewithin), such that each semi-circular retaining member locates with respect to spigots 49 extending laterally from the body of the middle component jig 41. Once the semi-circular retaining members 47 have been located with respect to the spigots 49, a gapped outer retaining member 51 is located over the semi-circular member to hold all the components together.

Next a plunger 53, as depicted in Fig. 14, is pushed into the open end of a void 55 defined by the semi-circular retaining members 47 and outer retaining member 51. The plunger 53, as shown in Fig. 14, comprises three rounded tongues 57 that are each separated from adjacent tongues by an interconnected generally Y-shaped slot 59.

As the plunger is pushed into the open end of the void, the tongues 57 drive the material of the middle scaffold component (which is wrapped around the material of the inner scaffold component) inwardly into the void 55 until the previously circular free end of the middle tubular component abuts against the valleys of the second component jig and locates in the slots 59 to form the edges of the generally Y-shaped line 29 (Fig. 8) along which the leaflets can separate to open the valve. In general, manually pushing the plunger into the void provides a force that is sufficient to deform the middle scaffold component to such an extent that the scaffold material retains its shape when the plunger is removed.

Once the plunger has been pushed into the void to deform the middle scaffold component, the outer retaining member 51 and semi-circular retaining members 47 can be removed. At this stage the middle scaffold component can be secured to the inner scaffold component, in particular along the edge of each leaflet 27 (corresponding to the edge of each trough 23), if so desired. Once the middle scaffold component has been secured to the inner scaffold component, the plunger can then be removed.

The next stage in the assembly process comprises wrapping a third sheet of biodegradable material around the middle scaffold component. This third sheet of material forms the outer scaffold component mentioned earlier, and adjacent edges of the third sheet are overlapped and secured one to the other (as described previously) to form a tube.

Next the outer component is secured to one or both of the inner and middle components, following which the jig assembly can be removed to leave an assembled valve scaffold that is ready for sterilisation, implantation and subsequent conditioning.

It will be appreciated from the above, that the provision of a jig set of the type described greatly facilitates the assembly of a biodegradable valve scaffold of the type described herein.

For example, the principles of a jig set for forming the valve scaffold may equally be applied to the formation of a vessel scaffold. In such a modification, the jig may be substantially simplified and may simply comprise a cylindrical body about which a first sheet and subsequently a second sheet of biodegradable material may be wrapped and secured.

It is also the case that whilst it is preferred for a jig assembly such as that depicted in Figs. 11 and 12 to be provided, it is conceivable that the inner tubual component may be formed directly on the jig depicted in Fig. 13 and subsequently cut to assume the crown-like shape of that jig. This modification has the advantage that it is not then necessary to remove the inner component from one jig component and transfer it to another.

In addition, whilst it is preferred for the middle component to be deformed in the manner described (namely by pushing a plunger into the void), it will be apparent that the middle component could be deformed by applying pressure laterally to deform the middle component against the jig to form the leaflets. In such a modification, neither the plunger nor the retaining members 47, 51 would need to be provided.

## Claims

1. A biodegradable scaffold for a prosthetic vessel or valve implant, the scaffold comprising: first and second tubular scaffold components co-axially arranged one inside the other and secured one to the other, each said tubular scaffold component being fabricable from a sheet of biodegradable material having two parallel peripheral edges that are overlapped and secured one to the other to form a tube, wherein the first and second tubular scaffold components are arranged such that the overlapped peripheral secured edges of said first tubular scaffold component and said second tubular scaffold component are at different positions about the circumference of the biodegradable scaffold.

2. A scaffold according to Claim 1, wherein the secured overlapped regions of the first and second tubes are arranged to be diametrically opposite one another about the circumference of said biodegradable scaffold.

3. A scaffold according to any preceding claim, wherein the overlapped peripheral edges of at least one said tubular scaffold component are:
a) sutured one to the other; or
b) sutured one to the other and joined one to the other by applying heat to weld one said edge to the other.

4. A scaffold according to Claim 3, wherein one said edge may be joined to the other along the entire length of the peripheral edges, or at one or more discrete locations.

5. A scaffold according to any of Claims 1 or 2, wherein the overlapped peripheral edges of each tubular scaffold component are joined by passing a member having a roughened outer surface through the overlapped peripheral edges, the roughened outer surface drawing fibres of one said overlapped edge into the other to knit the one to the other.

6. A scaffold according to any preceding claim, wherein said first and second tubular components are secured one to the other at a plurality of discrete locations about the periphery of the biodegradable scaffold.

7. A scaffold according to any preceding claim, further comprising a third tubular scaffold component coaxially arranged with and overlying said first and second tubular components, wherein said third tubular scaffold component is fabricable from a sheet of biodegradable material having two parallel peripheral edges that are overlapped and secured one to the other to form a tube, and is secured to at least one of said first and second tubular scaffold components.

8. A scaffold according to Claim 7, wherein said third tubular scaffold component is secured to at least one of said first and second tubular scaffold components at a plurality of discrete locations about the periphery of the biodegradable scaffold.

9. A scaffold according to Claim 8 when dependent on Claim 6, wherein the plurality of discrete locations at which said third tubular scaffold component is secured to at least one of said first and second tubular scaffold components is different to the plurality of discrete locations at which said first and second tubular components are secured one to the other.

10. The biodegradable scaffold according to claim 1, said biodegradable scaffold for a heart valve prosthesis, the scaffold comprising coaxially arranged inner, middle and outer tubular scaffold components each of which is fabricable from a sheet of biodegradable material having two parallel peripheral edges that are overlapped and secured one to the other to form a tube; wherein said inner scaffold component comprises a plurality of peaks at each of which the scaffold component has a larger longitudinal dimension in an axial direction, said peaks being separated from one another by trough regions wherein the scaffold component has a smaller longitudinal dimension in said axial direction, and a distal end of said middle scaffold component is shaped about said inner scaffold component to provide a plurality of valve leaflets each of which is coincident with respective ones of said troughs.

11. A method of assembling a biodegradable scaffold, the method comprising wrapping a first sheet of biodegradable material round a jig component, securing overlapped parallel ends of said first sheet together to form a first tubular scaffold component, wrapping a second sheet of biodegradable material round said first tubular scaffold component, and securing overlapped parallel ends of said second sheet together to form a second tubular scaffold component coaxially arranged with said first tubular component, wherein the first and second tubular scaffold components are arranged such that the overlapped peripheral secured edges of said first tubular scaffold component and said second tubular scaffold component are at different positions about the circumference of the biodegradable scaffold.

12. The method of claim 11, wherein said jig component has a crown-like shape consisting of a plurality of peaks at each of which the jig component has a larger longitudinal dimension in an axial direction, said peaks being separated from one another by trough regions, wherein the jig component has a smaller longitudinal dimension in said axial direction,
conforming said first sheet of material to the crown-like shape of said jig component; and
wrapping a second sheet of biodegradable material round said first tubular scaffold component.

13. A method according to Claim 12, wherein said first sheet of material is conformed to said crown-like shape by cutting said sheet, and said cutting is accomplished using said jig as a guide.

14. A method according to Claim 12 or 13, comprising the step of inwardly deforming a distal end region of said second tubular scaffold component to form a plurality of valve leaflets, each said leaflet being generally coincident with a said trough region, preferably -comprising - prior to said step of inwardly deforming the second tubular scaffold - the step of securing a retaining member having a generally circular cross section about the periphery of said second tubular component, wherein the inward deformation of said second tubular scaffold component is accomplished by pushing a shaped plunger into said retaining member to drive the distal end region of said second scaffold component against said jig.

## Patentansprüche

1. Biologisch abbaubares Gerüst für eine Gefäßprothese oder ein Klappenimplantat, wobei das Gerüst Folgendes umfasst: erste und zweite röhrenförmige Gerüstbestandteile, die koaxial ineinander angeordnet und aneinander befestigt sind, wobei jeder röhrenförmige Gerüstbestandteil aus einem Blatt aus biologisch abbaubarem Material mit zwei parallelen peripheren Rändern, die sich überlappen und zur Bildung einer Röhre aneinander befestigt sind, herstellbar ist, worin die ersten und zweiten röhrenförmigen Gerüstbestandteile so angeordnet sind, dass die überlappten peripheren befestigten Ränder des ersten röhrenförmigen Gerüstbestandteils und des zweiten röhrenförmigen Gerüstbestandteils an unterschiedlichen Stellen um den Umfang des biologisch abbaubaren Gerüsts angeordnet sind.

2. Gerüst nach Anspruch 1, worin die befestigten überlappten Regionen der ersten und zweiten Röhren so angeordnet sind, dass sie sich um den Umfang des biologisch abbaubaren Gerüsts diametral gegenüberliegen.

3. Gerüst nach einem der vorhergehenden Ansprüche, worin die überlappen peripheren Ränder des zumindest einen röhrenförmigen Gerüstbestandteils:
a) aneinander festgenäht sind; oder
b) aneinander festgenäht und durch Aufbringen von Wärme zum Verschweißen der Ränder miteinander miteinander verbunden sind.

4. Gerüst nach Anspruch 3, worin ein Rand über die gesamte Länge der peripheren Ränder oder an einer oder mehreren getrennten Stellen mit dem anderen Rand verbunden sein kann.

5. Gerüst nach einem der Ansprüche 1 oder 2, worin die überlappten peripheren Ränder jedes röhrenförmigen Gerüstbestandteils verbunden sind, indem ein Element mit einer aufgerauten Außenoberfläche durch die überlappten peripheren Ränder geführt wird, wobei die aufgeraute Außenoberfläche Fasern eines überlappten Rands in den anderen Rand zieht, um die beiden Ränder miteinander zu verwirken.

6. Gerüst nach einem der vorhergehenden Ansprüche, worin die ersten und zweiten röhrenförmigen Bestandteile an einer Vielzahl getrennter Stellen um die Peripherie des biologisch abbaubaren Gerüsts aneinander befestigt sind.

7. Gerüst nach einem der vorhergehenden Ansprüche, das ferner einen dritten röhrenförmigen Gerüstbestandteil umfasst, der koaxial zu den ersten und zweiten röhrenförmigen Bestandteilen angeordnet ist und über diesen liegt, worin der dritte röhrenförmige Gerüstbestandteil aus einem Blatt aus biologisch abbaubarem Material mit zwei parallelen peripheren Rändern, die sich überlappen und zur Bildung einer Röhre aneinander befestigt sind, herstellbar ist und zumindest an dem ersten oder dem zweiten röhrenförmigen Gerüstbestandteil befestigt ist.

8. Gerüst nach Anspruch 7, worin der dritte röhrenförmige Gerüstbestandteil zumindest an dem ersten oder an dem zweiten röhrenförmigen Gerüstbestandteil an einer Vielzahl getrennter Stellen um die Peripherie des biologisch abbaubaren Gerüsts befestigt ist.

9. Gerüst nach Anspruch 8 in Abhängigkeit von Anspruch 6, worin die Vielzahl getrennter Stellen, an denen der dritte röhrenförmigen Gerüstbestandteil zumindest an dem ersten oder an dem zweiten röhrenförmigen Gerüstbestandteil befestigt ist, sich von der Vielzahl getrennter Stellen unterscheiden, an denen die ersten und zweiten röhrenförmigen Bestandteile aneinander befestigt sind.

10. Biologisch abbaubares Gerüst nach Anspruch 1, wobei dieses biologisch abbaubare Gerüst für eine Herzklappenprothese bestimmt ist, worin das Gerüst koaxial angeordnete innere, mittlere und äußere röhrenförmige Gerüstbestandteile umfasst, die jeweils aus einem Blatt aus biologisch abbaubarem Material mit zwei parallelen peripheren Rändern, die sich überlappen und zur Bildung einer Röhre aneinander befestigt sind, herstellbar sind, worin der innere Gerüstbestandteil eine Vielzahl von Spitzen umfasst, an denen jeweils der Gerüstbestandteil eine größere Längsabmessung in axialer Richtung aufweist, wobei die Spitzen durch Talregionen voneinander getrennt sind, in denen der Gerüstbestandteil eine kleinere Längsabmessung in der axialen Richtung aufweist, und wobei ein distales Ende des mittleren Gerüstbestandteils um den inneren Gerüstbestandteil geformt ist, um eine Vielzahl von Klappensegeln bereitzustellen, die jeweils mit den jeweiligen Tälern zusammentreffen.

11. Verfahren zum Zusammensetzen eines biologisch abbaubaren Gerüsts, wobei bei dem Verfahren ein erstes Blatt aus biologisch abbaubarem Material um eine Spannvorrichtung gewickelt wird, die überlappten parallelen Enden des ersten Blattes zur Bildung eines ersten röhrenförmigen Gerüstbestandteils befestigt werden, ein zweites Blatt aus biologisch abbaubarem Material um den ersten röhrenförmigen Gerüstbestandteil gewickelt wird und die überlappten parallelen Enden des zweiten Blattes zur Bildung eines zweiten röhrenförmigen Gerüstbestandteils, der koaxial mit dem ersten röhrenförmigen Bestandteil angeordnet ist, aneinander befestigt werden, worin die ersten und zweiten röhrenförmigen Gerüstbestandteile so angeordnet sind, dass die überlappten peripheren befestigten Ränder des ersten röhrenförmigen Gerüstbestandteils und des zweiten röhrenförmigen Gerüstbestandteils an unterschiedlichen Stellen um den Umfang des biologisch abbaubaren Gerüsts angeordnet sind.

12. Verfahren nach Anspruch 11, worin die Spannvorrichtung eine kronenförmige Gestalt mit einer Vielzahl von Spitzen aufweist, wobei die Spannvorrichtung an jeder Spitze eine größere Längsabmessung in axialer Richtung aufweist, wobei die Spitzen durch Talregionen voneinander getrennt sind, worin die Spannvorrichtung in dieser axialen Richtung eine kleinere Längsabmessung aufweist, wobei das erste Materialblatt an die kronenförmige Gestalt der Spannvorrichtung angepasst wird; und wobei ein zweites Blatt aus biologisch abbaubarem Material um den ersten röhrenförmigen Gerüstbestandteil gewickelt wird.

13. Verfahren nach Anspruch 12, worin das erste Materialblatt durch Schneiden des Blattes an die kronenförmige Gestalt angepasst wird, und wobei das Schneiden mit der Spannvorrichtung als Führung erfolgt.

14. Verfahren nach Anspruch 12 oder 13, das den Schritt der Einwärtsverformung einer distalen Endregion des zweiten röhrenförmigen Gerüstbestandteils zur Bildung einer Vielzahl von Klappensegeln umfasst, wobei jedes Segel im Allgemeinen mit einer Talregion zusammentrifft, und vorzugweise - vor dem Schritt der Einwärtsverformung des zweiten röhrenförmigen Gerüsts - den Schritt der Befestigung eines Halteelements mit einem allgemein kreisförmigen Querschnitt um den Umfang des zweiten röhrenförmigen Bestandteils umfasst, worin die Einwärtsverformung des zweiten röhrenförmigen Gerüstbestandteils **dadurch** erfolgt, dass ein geformter Kolben in das Halteelement gedrückt wird, um die distale Endregion des zweiten Gerüstbestandteils gegen die Spannvorrichtung zu treiben.

## Revendications

1. Échafaudage biodégradable pour un implant de vaisseau ou de valvule prothétique, l'échafaudage comprenant : un premier et un deuxième composants d'échafaudage tubulaires disposés coaxialement l'un à l'intérieur de l'autre et solidement fixés l'un à l'autre, chacun desdits composants d'échafaudage tubulaires pouvant être fabriqué à partir d'une feuille de matériau biodégradable comportant deux bords périphériques parallèles que l'on fait se chevaucher et que l'on fixe solidement l'un à l'autre pour constituer un tube, dans lequel les premier et deuxième composants d'échafaudage tubulaires sont disposés de telle sorte que les bords périphériques, se chevauchant et solidement fixés, dudit premier composant d'échafaudage tubulaire et dudit deuxième composant d'échafaudage tubulaire soient à des endroits différents sur la circonférence de l'échafaudage biodégradable.

2. Échafaudage selon la revendication 1, dans lequel les zones de chevauchement et de fixation des premier et deuxième tubes sont disposées de sorte à être diamétralement opposées l'une à l'autre sur la circonférence dudit échafaudage biodégradable.

3. Échafaudage selon l'une quelconque des revendications précédentes, dans lequel les bords périphériques se chevauchant d'au moins un desdits composants d'échafaudage tubulaires sont :
a) suturés l'un à l'autre ; ou
b) suturés l'un à l'autre et assemblés l'un à l'autre par application de chaleur pour souder un desdits bords à l'autre.

4. Échafaudage selon la revendication 3, dans lequel un desdits bords peut être assemblé à l'autre sur toute la longueur des bords périphériques ou bien en un ou plusieurs endroits discrets.

5. Échafaudage selon l'une quelconque des revendications 1 et 2, dans lequel on assemble les bords périphériques se chevauchant de chaque composant d'échafaudage tubulaire en faisant passer un élément comportant une surface extérieure rendue rugueuse à travers les bords périphériques se chevauchant, la surface extérieure rendue rugueuse tirant des fibres d'un desdits bords se chevauchant dans l'autre pour les tricoter l'un à l'autre.

6. Échafaudage selon l'une quelconque des revendications précédentes, dans lequel lesdits premier et deuxième composants tubulaires sont solidement fixés l'un à l'autre en une pluralité d'endroits discrets autour de la périphérie de l'échafaudage biodégradable.

7. Échafaudage selon l'une quelconque des revendications précédentes, comprenant en outre un troisième composant d'échafaudage tubulaire disposé coaxialement avec lesdits premier et deuxième composants tubulaires et les recouvrant, dans lequel ledit troisième composant d'échafaudage tubulaire peut être fabriqué à partir d'une feuille de matériau biodégradable comportant deux bords périphériques parallèles, que l'on fait se chevaucher et que l'on fixe solidement l'un à l'autre pour constituer un tube, et est solidement fixé à au moins un desdits premier et deuxième composants d'échafaudage tubulaires.

8. Échafaudage selon la revendication 7, dans lequel ledit troisième composant d'échafaudage tubulaire est solidement fixé à au moins un desdits premier et deuxième composants d'échafaudage tubulaires en une pluralité d'endroits discrets autour de la périphérie de l'échafaudage biodégradable.

9. Échafaudage selon la revendication 8 quand elle est subordonnée à la revendication 6, dans lequel la pluralité d'endroits discrets où ledit troisième composant d'échafaudage tubulaire est solidement fixé à au moins un desdits premier et deuxième composants d'échafaudage tubulaires est différente de la pluralité d'endroits discrets où lesdits premier et deuxième composants tubulaires sont solidement fixés l'un à l'autre.

10. Échafaudage biodégradable selon la revendication 1, ledit échafaudage biodégradable pour une prothèse de valvule cardiaque lesdits composants comprenant des composants d'échafaudage tubulaires intérieur, intermédiaire et extérieur disposés coaxialement, composants pouvant chacun être fabriqués à partir d'une feuille de matériau biodégradable comportant deux bords périphériques parallèles que l'on fait se chevaucher et que l'on fixe solidement l'un à l'autre pour constituer un tube; et ledit composant d'échafaudage intérieur comprenant une pluralité de pics au niveau de chacun desquels le composant d'échafaudage a une plus grande dimension longitudinale dans une direction axiale, lesdits pics étant séparés les uns des autres par des zones de creux, où le composant d'échafaudage a une plus faible dimension longitudinale dans ladite direction axiale, et une extrémité distale dudit composant d'échafaudage intermédiaire est façonnée autour dudit composant d'échafaudage intérieur pour donner une pluralité de clapets de valvule, chacun desdits clapets coïncidant avec ceux correspondants desdits creux.

11. Procédé d'assemblage d'un échafaudage biodégradable, le procédé comprenant les opérations consistant à enrouler une première feuille de matériau biodégradable autour d'un composant de montage, fixer solidement l'une à l'autre les extrémités parallèles se chevauchant de ladite première feuille pour constituer un premier composant d'échafaudage tubulaire, enrouler une deuxième feuille de matériau biodégradable autour dudit premier composant d'échafaudage tubulaire et fixer solidement l'une à l'autre les extrémités parallèles se chevauchant de ladite deuxième feuille pour constituer un deuxième composant d'échafaudage tubulaire disposé coaxialement avec ledit premier composant tubulaire, les premier et deuxième composants d'échafaudage tubulaires étant disposés de telle sorte que les bords périphériques, se chevauchant et solidement fixés, dudit premier composant d'échafaudage tubulaire et dudit deuxième composant d'échafaudage tubulaire soient à des endroits différents sur la circonférence de l'échafaudage biodégradable.

12. Procédé selon la revendication 11, dans lequel ledit composant de montage a une forme du genre couronne consistant en une pluralité de pics au niveau de chacun desquels le composant de montage a une plus grande dimension longitudinale dans une direction axiale, lesdits pics étant séparés les uns des autres par des zones de creux, le composant de montage ayant une plus faible dimension longitudinale dans ladite direction axiale, le procédé comprenant les opérations consistant à :
conformer ladite première feuille de matériau sur la forme du genre couronne dudit composant de montage ; et
enrouler une deuxième feuille de matériau biodégradable autour dudit premier composant d'échafaudage tubulaire.

13. Procédé selon la revendication 12, dans lequel on conforme ladite première feuille de matériau sur la forme du genre couronne en découpant ladite feuille et ledit découpage s'effectue en utilisant ledit montage comme guide.

14. Procédé selon la revendication 12 ou 13, comprenant l'étape consistant à déformer vers l'intérieur une zone terminale distale dudit deuxième composant d'échafaudage tubulaire pour constituer une pluralité de clapets de valvule, chacun desdits clapets coïncidant dans l'ensemble avec une ladite zone de creux, de préférence comprenant - préalablement à l'étape consistant à déformer vers l'intérieur le deuxième composant d'échafaudage tubulaire - l'étape consistant à fixer solidement un élément de retenue de section de forme générale circulaire autour de la périphérie dudit deuxième composant tubulaire, la déformation vers l'intérieur dudit deuxième composant d'échafaudage tubulaire étant effectué en poussant un piston profilé dans ledit élément de retenue pour entrainer la zone terminale distale dudit deuxième composant d'échafaudage contre ledit montage.
